# EUROPÄISCHE PATENTANMELDUNG

(11) **EP 0 566 924 A2**
(43) Veröffentlichungstag der Anmeldung: **27.10.1993**
(21) Anmeldenummer: 93105620.4
(22) Anmeldetag: 05.04.1993
(51) Int. Cl.: C07C 45/38, C07C 45/39, B01J 23/50

(54) **Verfahren zur Herstellung von Dicarbonylverbindungen**

(30) Priorität: 21.04.1992 DE 4213008
(71) Anmelder: BASF Aktiengesellschaft, D-67063 Ludwigshafen (DE)
(72) Erfinder: Diercks, Rainer, Dr., W-6700 Ludwigshafen (DE); Weiser, Juergen, Dr., W-6905 Schriesheim (DE)

(57) **Zusammenfassung**

Herstellung von Dicarbonylverbindungen durch oxidative Dehydrierung von zweiwertigen primären oder sekundären Alkoholen in der Gasphase an einem Silber enthaltenden Katalysator, indem man die Reaktion in Gegenwart einer Phosphorverbindung vornimmt.

## Beschreibung

Die vorliegende Erfindung betrifft ein verbessertes Verfahren zur Herstellung von Dicarbonylverbindungen durch Dehydrierung von zweiwertigen primären oder sekundären Alkoholen in der Gasphase an einem Silber enthaltenden Katalysator in Gegenwart von Sauerstoff.

Die JP-A 86/22 039 lehrt die Herstellung von Oxybisaldehyden aus Glykolen durch katalytische oxidative Dehydrierung an silberhaltigen Katalysatoren.

Die Oxidation von Diethylenglykol zu 3-Oxa-pentandial an einem Silberkatalysator wird in der JP-A 87/277 334 beschrieben. Obwohl jeweils hohe Umsätze erzielt werden, sind diese Verfahren wegen der geringen Raum-Zeit-Ausbeute und der dadurch bedingten großen Apparateabmessungen für eine Produktion im industriellen Maßstab wirtschaftlich wenig attraktiv. So liegt die Raum-Zeit-Ausbeute im ersten Fall bei unter 1 kg Produkt pro Liter Katalysatorschüttvolumen und Stunde, im zweiten Fall unter 3 kg/l.h.

Nach der Lehre der EP-A 0 275 892 können Oligoalkylenglykolmonoaldehyde durch katalytische Oxydehydrierung der entsprechenden Alkohole an Silberkatalysatoren, die gegebenenfalls zusätzlich Phosphorverbindungen enthalten, hergestellt werden.

Aus der DE-A 40 22 603 ist bekannt, Methanol an einem Silber enthaltenden Katalysator in Gegenwart eines pulverförmigen, phosphorhaltigen Salzes, dessen Schmelz- oder Zersetzungstemperatur über der Reaktionstemperatur liegt, oxidativ zu Formaldehyd dehydrieren.

Es bestand die Aufgabe, ein Verfahren zur Verfügung zu stellen, das die selektive Herstellung von Dicarbonylverbindungen aus den entsprechenden zweiwertigen Alkoholen bei hohen Umsätzen erlaubt. Weiterhin soll das Verfahren eine hohe Raum-Zeit-Ausbeute aufweisen.

Demgemäß wurde eine Verbesserung des eingangs definierten Verfahrens gefunden, die dadurch gekennzeichnet ist, daß man die Reaktion in Gegenwart von Phosphorverbindungen vornimmt.

Das erfindungsgemäße Verfahren läßt sich mit solchen primären und sekundären Alkoholen ausführen, die ohne merkliche Zersetzung verdampfbar sind. Besondere Bedeutung hat das Verfahren für die Herstellung von Dicarbonylverbindungen der allgemeinen Formel I 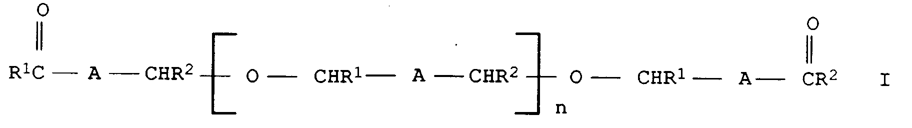
in der die Variablen folgende Bedeutung haben:
- R¹, R²: Wasserstoff, C₁-C₈-Alkyl, Phenyl oder Benzyl
- n: 0 bis 4
- A: chemische Bindung oder Alkylenrest mit bis zu 3 Kohlenstoffatomen
aus Oligoalkylenglykolen der allgemeinen Formel II 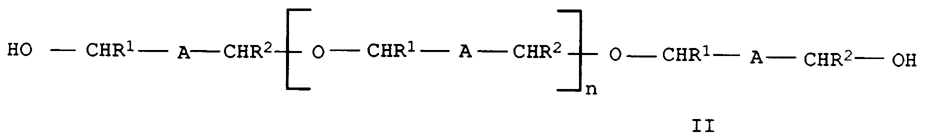
Die Ausgangsverbindungen II sind bekannt oder nach bekannten Methoden herstellbar. Es kommen vor allem solche Oligoalkylenglykole in Betracht, die durch Oligomerisierung von Ethylenoxid, 1,2-Propylenoxid, 1,2-Butylenoxid und Tetrahydrofuran erhalten werden. Besonders bevorzugt ist Diethylenglykol.

Daneben kommen weiterhin aliphatische Diole wie 1,4-Butandiol, Pentandiole oder Hexandiole in Betracht.

Die Ausgangsverbindungen werden gasförmig über den Katalysator geleitet. Als Oxidationsmittel wird Sauerstoff verwendet. Dieser kann in reiner Form, bevorzugt aber verdünnt mit inerten Gasen wie Edelgasen, Stickstoff oder Kohlenmonoxid zugegeben werden. Besonders vorteilhaft ist die Verwendung von Luft, die man im Gemisch mit den zu oxidierenden gasförmigen Diolen einsetzen kann. In der Regel werden 0,6-1,0 mol Sauerstoff pro mol Diol eingesetzt, vorteilhaft 0,7-0,9 mol pro mol Diol.

Es hat sich als günstig erwiesen, dem Ausgangsgemisch Wasserdampf zuzusetzen, wobei insbesondere 0,1 bis 15 mol Wasser, bevorzugt 1 bis 8 mol, besonders bevorzugt 2 bis 6 mol Wasser pro Mol Diol verwendet werden.

Die Oxidation zu den Dicarbonylverbindungen findet an einem wirksame Mengen Silber enthaltenden Katalysator statt. So kann Silber auf Trägern wie Aluminiumoxid, Siliciumdioxid, Titandioxid, Silicaten oder Mischungen davon eingesetzt werden. Weiterhin kann Silber in Form von Legierungen wie Silber-Gold- oder Silber-Kupfer-Legierung als Katalysator fungieren. Vorzugsweise wird aber Silber elementar, z.B. in Form eines Netzes oder besonders vorteilhaft in Form von Silberkörnern verwendet. In einer besonders bevorzugten Ausführungsform werden Mischungen verschieden großer Silberkörner eingesetzt, die Abmessungen von 0,2 bis 1 mm Durchmesser haben.

Je nach dem Silbergehalt des Katalysators liegt die Katalysatorbelastung im allgemeinen bei 1 bis 200 kg Diol/kg Katalysator·h, bevorzugt aber bei 5 bis 100 kg Diol/kg·h.

Erfindungsgemäß wird die Oxidation in Gegenwart von Phosporverbindungen durchgeführt. Als Phosphorquelle können flüchtige organische Phosphorverbindungen wie Phosphite, Phosphane, beispielsweise Triphenylphosphan, oder Phosphorsäureester wie Trimethylphosphat dienen. Diese Verbindungen können mit dem Ausgangsgasgemisch aus Diol und Sauerstoff dem Katalysator zugeführt werden. Als verfahrenstechnisch günstiger hat sich aber erwiesen, Phosphorsalze auf dem Katalysator aufzubringen, sei es z.B. durch Aufstreuen oder durch Abscheiden aus Lösungen. Dafür kommen vor allem Phosphate oder Polyphosphate in Betracht.

Bevorzugt werden Alkalimetall- oder Erdalkalimetallphosphate oder -pyrophosphate, z.B. Na₄P₂O₇, Li₃PO₄, Mg₃(PO₄)₂ und Ca₃(PO₄)₂. Die Menge der eingesetzten Verbindungen liegt im allgemeinen bei 0,05 bis 100 mg Phosphor pro Gramm Katalysator, bevorzugt 5 bis 50 mg.

Die Oxidation kann in einem weiten Temperaturbereich von etwa 200 bis 900°C erfolgen. Vorteilhaft sind jedoch 400 bis 800°C, wobei der Bereich von 550 bis 750°C besonders bevorzugt wird.

Der bei der Reaktion herrschende Druck ist nicht kritisch. Die Reaktion wird daher in der Regel bei Normaldruck vorgenommen. Es kann jedoch vorteilhaft sein, bei vermindertem Druck bis etwa 0,1 bar zu arbeiten, wenn die Ausgangsdiole schwer verdampfbar sind.

Das die Reaktionszone verlassende Produktgemisch wird zweckmäßigerweise abgekühlt und kondensiert. Je nach zugesetzter Wassermenge werden wäßrige Lösungen der jeweiligen Dicarbonylverbindung erhalten, die gegebenenfalls weiter aufgearbeitet werden können.

Das erfindungsgemäße Verfahren überführt primäre oder sekundäre zweiwertige Alkohole in hoher Selektivität bei fast vollständigem Umsatz in die entsprechenden Dicarbonylverbindungen, wobei eine hohe Raum-Zeit-Ausbeute erreicht wird.

Die Verfahrensprodukte sind wertvolle Zwischenprodukte. Sie eignen sich als Gerbstoffe für Leder und Felle.

### Beispiel

### Erfindungsgemäße Herstellung von 3-Oxa-pentan-1,5-dial

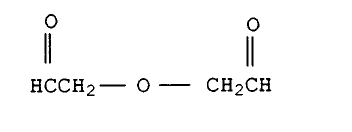

Eine Schüttung aus 4 g Silberkörnern mit 0,75 mm bis 1 mm Durchmesser, 7 g mit 0,4 mm bis 0,75 mm und 3 g mit 0,2 mm bis 0,4 mm (Schüttvolumen 3,14 ml) wurden mit 72 mg Na₄P₂O₇ überstreut (17 mg Phosphor pro Gramm Katalysator). 313 g Diethylenglykol wurden pro Stunde verdampft, mit Wasserdampf im Verhältnis von 4 mol Wasser zu 1 mol Diol gemischt und mit Luft (0,8 mol Sauerstoff pro mol Diol) bei 705°C umgesetzt (Katalystorbelastung 22 kg Diol/kg.h). Bei einem Umsatz von 98 % betrug die Selektivität 70 %, woraus sich eine Ausbeute von 69 % ergibt.

Die Raum-Zeit-Ausbeute betrug 66,2 kg Produkt/l.h.

### Vergleichsbeispiel

Zum Vergleich wurde wie im obigen Beispiel, jedoch ohne Phosphorzusatz, gearbeitet. Außerdem wurde die Temperatur auf 675°C gesenkt, da sonst die Selektivität erheblich niedriger war. Es wurden 0,75 mol Sauerstoff pro mol Diol eingesetzt.

Der Umsatz betrug 98 %, die Selektivität 60 %. Die Ausbeute betrug somit 59 %.

Die Raum-Zeit-Ausbeute betrug 56,6 kg/l.h.

## Patentansprüche

1. Verfahren zur Herstellung von Dicarbonylverbindungen durch Dehydrierung von zweiwertigen primären oder sekundären Alkoholen in der Gasphase an einem Silber enthaltenden Katalysator in Gegenwart von Sauerstoff, dadurch gekennzeichnet, daß man die Reaktion in Gegenwart einer Phosphorverbindung vornimmt.

2. Verfahren nach Anspruch 1, dadurch gekennzeichnet, daß die Phosphormenge 0,05 bis 100 mg pro Gramm Katalysator beträgt.

3. Verfahren nach Anspruch 1 oder 2, dadurch gekennzeichnet, daß man die Reaktion bei 550 bis 750°C vornimmt.

4. Verfahren nach den Ansprüchen 1 bis 3, dadurch gekennzeichnet, daß man Dicarbonylverbindungen der allgemeinen Formel I 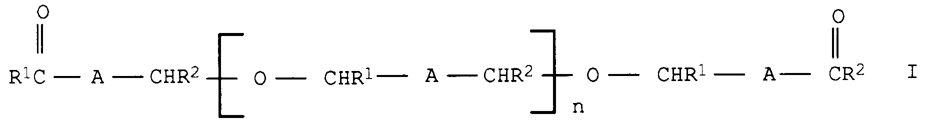 in der die Variablen folgende Bedeutung haben:
R¹, R² Wasserstoff, C₁-C₈-Alkyl, Phenyl oder Benzyl
n 0 bis 4
A chemische Bindung oder Alkylenrest mit bis zu 3 Kohlenstoffatomen
aus Oligoalkylenglykolen der allgemeinen Formel II 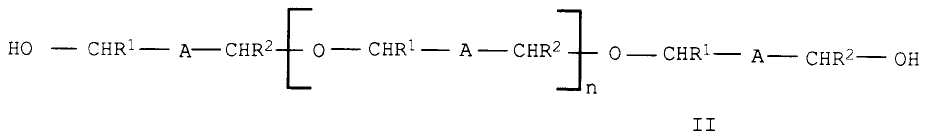 herstellt.

5. Verfahren nach den Ansprüchen 1 bis 4, dadurch gekennzeichnet, daß man Diethylenglykol oxidativ zu 3-Oxa-pentandial dehydriert.
